# EUROPEAN PATENT SPECIFICATION

(11) **EP 3 831 341 B1**
(45) Date of publication and mention of the grant of the patent: **21.10.2026**
(21) Application number: 20216184.0
(22) Date of filing: 16.09.2013
(51) Int. Cl.: A61F 2/02, A61F 2/12, A61F 2/52

(54) **BREAST IMPLANTS WITH INTEGRATED TRANSPONDERS**
BRUSTIMPLANTATE MIT INTEGRIERTEN TRANSPONDERN
IMPLANTS MAMMAIRES À TRANSPONDEURS INTÉGRÉS

(30) Priority: 17.09.2012 US 201261701910 P
(43) Date of publication of application: 09.06.2021
(62) Document number(s) of the earlier application(s) in accordance with Art. 76 EPC: 18210589.0 / 3 479 796; 13839796.3 / 2 895 109
(73) Proprietor: JAMM Technologies, Inc., 20113 Alajuela (CR)
(72) Inventor: MEJIA, Ezequiel, Woodbury, MN 22125 (US); GEISSLER, Randolph Keith, Hudson, WI 54016 (US); CHACÓN QUIRÓS, Juan José, 20113 Alajuela (CR)
(74) Representative: Haseltine Lake Kempner LLP

(56) References cited:
- WO-A2-2010/056610

## Description

### RELATED APPLICATIONS

This application claims the benefit of, and priority to, U.S. Provisional Application No. 61/701,910, filed September 17, 2012, entitled "Breast Implants With Integrated Transponders,"

### BACKGROUND

Currently there is no accurate, adequate, practical or economical way to identify a breast implant that is already inside the body of a patient. This void has developed into a serious complication for the fulfillment of recalls and controls, when the patients do not have the identification information for their implants, or when medical records are not available. More specifically, and relating to defective breast implants which have been already found in the market, many women around the world face the problem of having no information regarding the breast implants inside them, and no way of finding out if they need to be explanted or not, without undergoing an actual explantation procedure. Thus, there is a need in the art for breast implants that, when implanted, can be easily and accurately identified from outside the body. WO 2010/056610 A2 discloses the manufacturing of a soft prosthetic implant by providing fluid dispersion, molding fluid dispersion to form hollow shell having internal cavity, and assembling secondary component with shell to form implant.

### SUMMARY

The present invention relates to a breast implant designed to add to or replace volume of the breast, which includes a unique device identifier (UDI) therein, providing post-implantation device recognition and traceability. In preferred embodiments, the UDI is a passive RFID (radio frequency identification) transponder, which is embedded within the implant filling at the time of manufacture.

In various embodiments, the present invention provides breast implant comprising a silicone elastomer shell with an opening covered by a patch, a silicone gel filling said shell, and a transponder disposed within said filling and positioned proximal to said patch.

The silicone elastomer shell is formed by preparing a silicone elastomer dispersion; dipping a mold into said dispersion one or more times, forming one or more silicone elastomer layers; and removing the one or more layers from the mold, forming a silicone elastomer shell having an opening where the mold was removed.

In some embodiments, the transponder is placed within the shell through said opening. Said opening is then covered with a patch and the patched shell is filled with a silicone gel through a filling hole in the patch.

In other embodiments, said opening is covered with a patch, the patched shell is filled with a silicone gel through a filling hole in the patch, and the transponder is placed within the patched shell through said filling hole.

In still further embodiments, said opening is covered with a patch, the transponder is placed within the patched shell through a filling hole in the patch, and the patched shell is filled with a silicone gel through said filling hole.

The manufacturing process further includes applying a vacuum to the silicone gel-filled shell, removing air bubbles and positioning the transponder proximal to said patch; sealing the filling hole; and curing the silicone gel, wherein said gel curing further positions the transponder proximal to said patch and comprises heating the silicone-gel filled shell with transponder therein to a temperature of about 140 °C to 200 °C for up to about 8 hours.

In some embodiments, the breast implant with integrated transponder is further sterilized at a temperature of about 120 °C to 150 °C for up to about 36 hours.

Additional features and advantages of the present invention are described further below. The invention relates to a breast implant as defined in claim 1, Embodiments of the invention are recited in the dependent claims.

### BRIEF DESCRIPTION OF THE DRAWINGS

The foregoing summary, as well as the following detailed description of the preferred embodiments of the application, will be better understood when read in conjunction with the appended drawings. For the purposes of illustrating the device of the present application, there are shown in the drawings preferred embodiments. It should be understood, however, that the application is not limited to the precise arrangements and instrumentalities shown. In the drawings:
FIG. 1 shows a flowchart of exemplary processes involved in manufacturing the breast implants of the present invention, according to some embodiments.
FIG. 2 shows a schematic diagram of breast implants of the present invention, according to some embodiments, illustrating exemplary placement of a microchip/transponder.

### DETAILED DESCRIPTION

The present invention provides, in various embodiments, a breast implant with an RFID transponder embedded therein, so that the implant can be conveniently identified while inside the human body, and methods of making the same. The transponder contains a unique identification code and/or implant-specific information that is readily accessible, for example, by an external handheld scanner. In some embodiments, identification information from the transponder can be used to access one or more databases containing further information (regarding the specific breast implant, the specific patient, etc.).

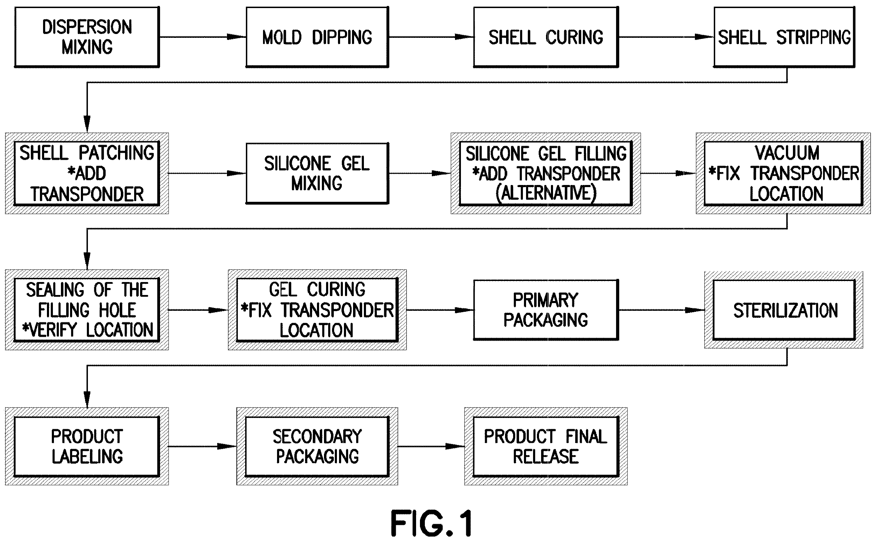

FIG. 1 shows an exemplary flowchart of the different processes involved in the manufacturing of a breast implant of the present invention, according to some embodiments. The processes affected by inclusion of a transponder are highlighted, and points where the transponder is added/fixed/verified are marked with an asterisk.

The breast implant comprises a silicone elastomer shell. As shown in FIG. 1, this shell may be formed by preparing/mixing a silicone elastomer dispersion, and dipping a mold or mandrel into the dispersion. Multiple dip coats may be used to obtain a multilayered shell, and different dispersions may be used for different dip coats. For example, a colored barrier layer may be added to the shell, as described in U.S. Application No. 13/412,221. The shell is then cured and removed/stripped from the mold.

The aperture (open part of the shell where the mold was removed) is then patched, for example, with a piece of silicone elastomer similar to the cured shell. In some embodiments, the transponder is introduced into the breast implant during the shell patching (through the opening in the shell before the shell is patched). The patch is preferably secured by high pressure and heat. Alternatively, an adhesive or other method may be used to secure the patch. The patched silicone elastomer shell may be dried and cured, and is then filled (e.g., by a syringe) through a hole in the patch system (including the area where the patch joins with the shell) to a predetermined weight with fluid or gel. In some embodiments, the transponder is added during the silicone gel filling, through the hole used for the silicone gel filling. As shown in FIG. 1, in some embodiments, a highly viscous and highly elastic silicone gel is mixed and used (uncured) for filling the breast implant. The silicone gel may include a platinum catalyst. The filled implant is then placed in a vacuum chamber, where it undergoes one or more cycles of vacuum. If there are any bubbles still visible, additional vacuum cycles may be added.

The hole through which the implant was filled is then sealed (e.g., with an RTV silicone adhesive), and the silicone gel is cured. Preferably, the gel curing comprises high temperature curing (e.g., about 160 °C). In some embodiments, the implant may be filled with liquid or alternate fillers, which do not need further curing or vacuum cycles. The breast implant may then be cooled down to room temperature and placed in primary packaging (e.g., a double pack comprising a pair of implants). Sterilization may then be performed. Sterilization is preferably performed using dry heat (e.g., about 120 to 130 °C for about 24 to 48 hours). Alternatively, the implant may be sterilized using other sterilization methods, such as ethylene oxide.

Product labeling and secondary packaging processes are then performed before the final release of the product. In some embodiments, for example, product labeling assigns a serial number to the implant, and stores the serial number with information about the associated implant in a computer database (e.g., in an ERP system). Secondary packaging assigns the transponder identification code (e.g., 16 digit code) with the serial number of the implant. Product final release verifies that the transponder identification code matches the serial number of the implant. In other embodiments, different labeling/packaging processes may be used, as long as they match the transponder identification number to the serial number of each device.

Notably, Applicant has determined that the placement of the transponder within the breast implant is important for safe and effective function of the invention. For example, in some embodiments, the transponder may be affixed to the breast implant shell. However, the transponder is fragile and could break and/or could puncture the implant shell, thus it is preferred to place the transponder in a stronger part of the product. Accordingly, in other, more preferred embodiments, the transponder is suspended within the implant filling, close to the patch.

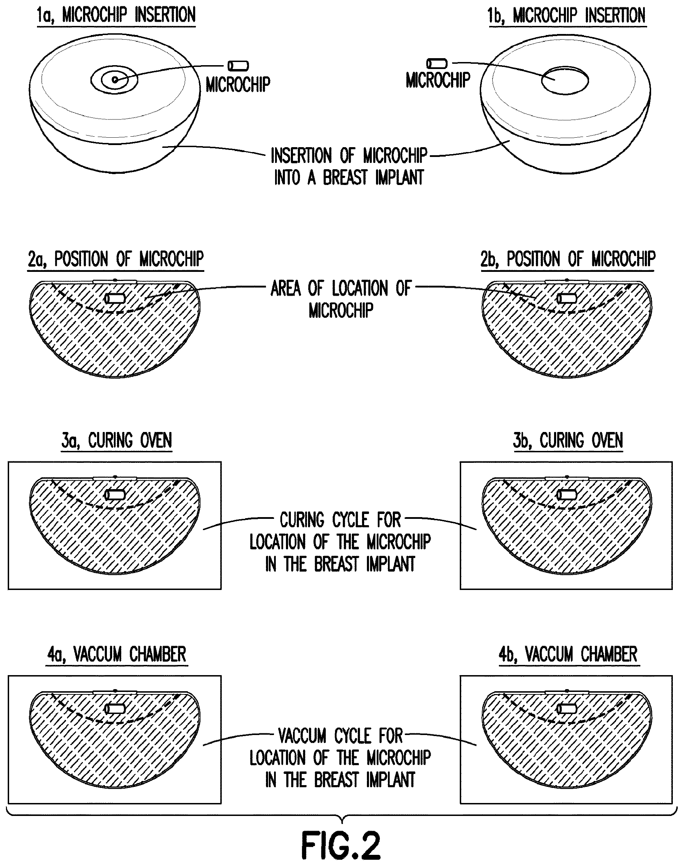

As indicated in FIG. 1, the transponder (microchip) can be added to the implant at different parts of the manufacturing process. For example, as shown in FIG. 2, the transponder can be incorporated during the Shell Patching (through the opening in the shell before patching; lb). Alternatively, the transponder can be incorporated during the Silicone Gel Filling (through the hole in the patch system used for silicone gel filling; la).

In preferred embodiments, the transponder in the silicone gel is free floating, but in close proximity to the patch, which is at the back side of the implant (FIG. 2; 2a, 2b). Accordingly, once implanted, the transponder will be farthest from the exterior of the patient, which can protect the transponder and also ensure that it will not be felt (e.g., under the patient's skin when touched). The density of the silicone gel mixed for the implant filling is thus chosen so that the: transponder will float in the silicone gel near the surface (the patch, area). The transponder placement in the silicone filling gel at the back of the patch system is adjusted/fixed during the vacuum process (FIG. 2; 4a, 4b), and is verified when the filling hole is sealed.

Preferably, the transponder is added before the silicone gel is cured, so that is secured by the silicone. If the transponder is added after curing, it will break the gel when it is inserted and will not be well-integrated. The transponder placement in the silicone gel near the patch is further adjusted/fixed during the gel curing process (FIG. 2; 3a, 3b). Advantageously, when the gel is cured with the transponder embedded therein, the transponder is flexibly fixed in the gel, and like a rubber band will come back to the same location even when the implant is manipulated/deformed and the transponder is moved therein.

As described above and shown in FIG. 1, several breast implant manufacturing steps involve high temperatures, and high and low pressures. For example, gel curing may use a temperature up to about 200 °C (e.g., between 120 °C and 190 °C) for up to 8 hours. Sterilization may use a temperature up to about 150 °C (e.g., between 110 °C and 145 °C) for up to 48 hours. Transponders integrated within the implants as described herein, must be able to withstand such manufacturing conditions.

Advantageously, the present invention uses a high temperature-stable glass encapsulated transponder, which can withstand temperatures up to 210 °C (± 20 °C) and can be integrated into the body of the breast implant during the manufacture of the implant without loss of function thereafter. Such transponders are described in co-pending U.S. Application No. 14/027,896, entitled "High Temperature Transponders," . In addition to their resistance to heat changes, these transponders provide good shock resistance, long term reliability, long range sensitivity, and small size, among other advantages.

While there have been shown and described fundamental novel features of the invention as applied to the preferred and exemplary embodiments thereof, it will be understood that omissions and substitutions and changes in the form and details of the disclosed invention may be made by those skilled in the art without departing from the scope of the invention. Moreover, as is readily apparent, numerous modifications and changes may readily occur to those skilled in the art. It is the intention, therefore, to be limited only as indicated by the scope of the claims appended hereto.

## Claims

1. A breast implant, comprising:
a multilayered silicone shell;
a patch covering an opening of the shell;
a silicone gel disposed in the shell; and
a radio frequency identification (RFID) transponder suspended in the silicone gel and positioned proximal to the patch, wherein the transponder is an RFID transponder that contains a unique identification code.

2. The breast implant of claim 1, wherein the implant is enclosed in packaging, and the packaging associates a serial number of the implant with the identification code of the transponder, the serial number being stored in a computer database.

3. The breast implant of claim 1 or 2, wherein the shell includes a colored barrier layer.

4. The breast implant of any one of claims 1-3, wherein the transponder is a high temperature-stable glass encapsulated transponder.

5. The breast implant of any one of claims 1-4, wherein the transponder is capable of withstanding a temperature of 210°C ± 20 °C without loss of function thereafter.

6. The breast implant of any one of claims 1-5, wherein the patch is secured to the shell by pressure and heat.

7. The breast implant of any one of claims 1-5, wherein the patch is sealed to the shell with an adhesive.

## Patentansprüche

1. Brustimplantat, umfassend:
eine mehrschichtige Silikonhülle;
ein Pflaster, das eine Öffnung der Hülle abdeckt;
ein in der Hülle angeordnetes Silikongel; und
einen Radiofrequenzidentifikations-Transponder (RFID-Transponder), der in dem Silikongel suspendiert und proximal zu dem Pflaster positioniert ist, wobei es sich bei dem Transponder um einen RFID-Transponder handelt, der einen eindeutigen Identifikationscode enthält.

2. Brustimplantat nach Anspruch 1, wobei das Implantat in einer Verpackung eingeschlossen ist und die Verpackung eine Seriennummer des Implantats mit dem Identifikationscode des Transponders verknüpft, wobei die Seriennummer in einer Computerdatenbank gespeichert ist.

3. Brustimplantat nach Anspruch 1 oder 2, wobei die Hülle eine farbige Barriereschicht aufweist.

4. Brustimplantat nach einem der Ansprüche 1-3, wobei es sich bei dem Transponder um einen hochtemperaturstabilen, in Glas eingekapselten Transponder handelt.

5. Brustimplantat nach einem der Ansprüche 1-4, wobei der Transponder in der Lage ist, einer Temperatur von 210 °C ± 20 °C ohne anschließenden Funktionsverlust standzuhalten.

6. Brustimplantat nach einem der Ansprüche 1-5, wobei das Pflaster durch Druck und Hitze an der Hülle befestigt ist.

7. Brustimplantat nach einem der Ansprüche 1-5, wobei das Pflaster mit einem Klebstoff an der Hülle abgedichtet ist.

## Revendications

1. Implant mammaire, comprenant :
une coque en silicone multicouche ;
une pastille recouvrant une ouverture de la coque ;
un gel de silicone disposé dans la coque ; et
un transpondeur d'identification par radiofréquence (RFID) suspendu dans le gel de silicone et positionné à proximité de la pastille, dans lequel le transpondeur est un transpondeur RFID qui contient un code d'identification unique.

2. Implant mammaire selon la revendication 1, dans lequel l'implant est enfermé dans un emballage, et l'emballage associe un numéro de série de l'implant au code d'identification du transpondeur, le numéro de série étant stocké dans une base de données informatique.

3. Implant mammaire selon la revendication 1 ou 2, dans lequel la coque comporte une couche barrière colorée.

4. Implant mammaire selon l'une quelconque des revendications 1 à 3, dans lequel le transpondeur est un transpondeur encapsulé dans du verre stable aux hautes températures.

5. Implant mammaire selon l'une quelconque des revendications 1 à 4, dans lequel le transpondeur est capable de résister à une température de 210 °C ± 20 °C sans perte ultérieure de fonctionnalité.

6. Implant mammaire selon l'une quelconque des revendications 1 à 5, dans lequel la pastille est fixée à la coque par pression et chaleur.

7. Implant mammaire selon l'une quelconque des revendications 1 à 5, dans lequel la pastille est scellée sur la coque au moyen d'un adhésif.
